(19) Europäisches Patentamt
European Patent Office
Office européen des brevets

(11) Veröffentlichungsnummer: **0 019 587**
**B1**

# (12) EUROPÄISCHE PATENTSCHRIFT

(45) Veröffentlichungstag der Patentschrift: **20.07.83**

(51) Int. Cl.³: **C 07 C 149/40**, A 61 K 31/19, A 61 K 31/22

(21) Anmeldenummer: **80810152.1**

(22) Anmeldetag: **06.05.80**

## (54) Neue Carbonsäurehydrazide, Verfahren zu ihrer Herstellung, ihre Verwendung und sie enthaltende pharmazeutische Zusammensetzungen.

(30) Priorität: **11.05.79 CH 4416/79**

(43) Veröffentlichungstag der Anmeldung: **26.11.80 Patentblatt 80/24**

(45) Bekanntmachung des Hinweises auf die Patenterteilung: **20.07.83 Patentblatt 83/29**

(84) Benannte Vertragsstaaten: **CH DE FR GB IT LI**

(56) Entgegenhaltungen:
**DE-A-1 493 797**
**DE-A-2 023 415**

(73) Patentinhaber: **CIBA-GEIGY AG, Patentabteilung Postfach, CH-4002 Basel (CH)**

(72) Erfinder: **Sallmann, Alfred, Dr., Joachimsackerstrasse 12, CH-4103 Bottmingen (CH)**
Erfinder: **Göschke, Richard, Dr., Felixhäglistrasse 21, CH-4103 Bottmingen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 587 B1

ACTORUM AG

# Neue Carbonsäurehydrazide, Verfahren zu ihrer Herstellung, ihre Verwendung und sie enthaltende pharmazeutische Zusammensetzungen.

Die Erfindung betrifft neue Carbonsäurehydrazide der Formel

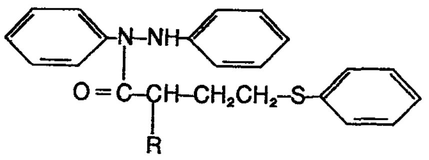

(I),

worin R eine gegebenenfalls mit einem Alkohol aliphatischen Charakters veresterte Carboxygruppe bedeutet, und pharmazeutisch verwendbare Salze der Verbindung der Formel I, worin R Carboxy ist, mit Basen, Verfahren zur Herstellung der neuen Verbindungen, diese enthaltende pharmazeutische Präparate und ihre Verwendung als Arzneimittelwirkstoffe.

Mit einem Alkohol aliphatischen Charakters verestertes Carboxy R ist beispielsweise gegebenenfalls substituiertes Niederalkoxycarbonyl, z.B. Niederalkoxycarbonyl, Hydroxy- bzw. Niederalkoxyniederalkoxycarbonyl oder gegebenenfalls im Phenylteil, z.B. durch Niederalkyl, Niederalkoxy, Halogen und/oder Trifluormethyl, substituiertes Phenylniederalkoxycarbonyl.

Unter «niederen» organischen Resten und Verbindungen sind solche zu verstehen, die bis zu 7, vor allem bis zu 4, C-Atome aufweisen. Ferner gilt:

Niederalkoxycarbonyl ist beispielsweise Methoxy-, Äthoxy-, Propyloxy-, Isopropyloxy- oder Butyloxy-, Isobutyloxy-, Sekundärbutyloxy- oder Tertiärbutyloxycarbonyl, ferner Pentyloxy-, Hexyloxy- oder Heptyloxycarbonyl.

Hydroxyniederalkoxycarbonyl kann 1 oder 2 Hydroxygruppen aufweisen und ist vorzugsweise solches, in dem die Hydroxygruppe(n) in höherer als der 1-Stellung gebunden ist (sind), beispielsweise 2-Hydroxyäthoxy-, 2- oder 3-Hydroxypropyloxy- oder 2,3-Dihydroxypropyloxycarbonyl.

Niederalkoxyniederalkoxycarbonyl ist beispielsweise solches, in dem die terminale Niederalkoxygruppe in höherer als der 1-Stellung gebunden ist, beispielsweise 2-Methoxy-oder 2-Äthoxyäthoxycarbonyl oder 3-Methoxy- oder 3-Äthoxypropyloxycarbonyl.

Niederalkyl ist beispielsweise Methyl, Äthyl, Propyl, Isopropyl, Butyl, Sekundärbutyl, Isobutyl oder Tertiärbutyl, ferner Pentyl, Hexyl oder Heptyl.

Niederalkoxy ist beispielsweise Methoxy, Äthoxy, Propyloxy, Isopropyloxy, Butyloxy, Isobutyloxy, Sekundärbutyloxy oder Tertiärbutyloxy, ferner Pentyloxy, Hexyloxy oder Heptyloxy.

Halogen ist beispielsweise Halogen bis und mit Atomnummer 35, wie Fluor, Chlor oder Brom.

Phenylniederalkoxycarbonyl ist beispielsweise Benzyloxycarbonyl, 2-Phenyläthoxycarbonyl oder 3-Phenylpropyloxycarbonyl.

Pharmazeutisch verwendbare Salze der Verbindung der Formel I, worin R Carboxy ist, mit Basen sind beispielsweise pharmazeutisch verwendbare Metall- oder Ammoniumsalze. Pharmazeutisch verwendbare Metallsalze sind beispielsweise Alkali- oder Erdalkalimetallsalze, z.B. Lithium-, Natrium-, Kalium-, Magnesium- oder Calciumsalze, ferner Zink-, Eisen- oder Kupfersalze. Pharmazeutisch verwendbare Ammoniumsalze sind beispielsweise Ammoniumsalze mit Ammoniak oder Aminen, wie gegebenenfalls C-substituierten, z.B. C-hydroxylierten oder C-aminierten, Niederalkylaminen, z.B. mit Di- oder Triäthylamin, Dimethylamin, 2-Hydroxy-1,1-bis(hydroxymethyl)-äthylamin, Triäthanolamin, Äthylendiamin oder Guanidin, ferner Ammoniumsalze mit cyclischen organischen Aminen, wie Piperidin, Piperazin, N'-Methyl-piperazin oder Morpholin.

Die Verbindung der Formel I und ihre pharmazeutisch verwendbaren Salze besitzen wertvolle pharmazeutische Eigenschaften. Pharmazeutisch verwendbare Carbonsäurehydrazide sind bereits bekannt. So sind in der DE-A-2 023 415 der Formel I entsprechende, urikosurisch wirksame Säuren und Säuresalze, die anstelle der β-Phenylthioäthylgruppe β-Phenylsulfinyläthyl aufweisen, und in der DE-A-1 493 797 in den Anilinteilen gegebenenfalls substituierte, der Formel I entsprechende, antiphlogistisch und antipyretisch wirksame Säuren und Salze, die anstelle der Phenylthiogruppe «einen Kohlenwasserstoffrest mit höchstens 8 Kohlenstoffatomen» aufweisen, beschrieben. Die anmeldungsgemässen Verbindungen weisen andersartige pharmakologische Eigenschaften auf. So hat sich herausgestellt, dass sie in Versuchsanordnungen zum Nachweis einer antithrombotischen Wirkung, insbesondere in Cyclooxygenase-abhängigen Testen, eine ausgeprägte antithrombotische Aktivität besitzen. Diese kann z.B. in vivo anhand der Arthus-Reaktion, siehe dazu Brit. J. Pharmacology, 57, 441 p (1976), im Dosisbereich von etwa 5–100 mg/kg p.o. und am Kaninchen anhand ihrer Hemmwirkung auf die durch Arachindonsäure induzierte Lungenembolie, siehe dazu Pharmacology, 14, 522 (1976), im Dosisbereich von etwa 0,3 bis 20 mg/kg p.o. sowie in vitro anhand der Hemmung der Prostaglandin-Synthese aus Arachidonsäure durch Prostaglandin-Synthetease, nachgewiesen in der Versuchsanordnung nach Prostaglandins 7, 123 (1974) im Konzentrationsbereich von 75–1500 mg/l, dokumentiert werden.

Die Verbindungen der Formel I und ihre pharmazeutisch verwendbaren Salze sind dementsprechend ausgezeichnet geeignet zur Behandlung cardiovaskulärer Erkrankungen, insbesondere zur Behandlung thrombotischer Erkrankungen, vor allem von Zuständen, bei denen eine abnorme Funktion der Blutplättchen den verursachenden oder einen begleitenden Faktor darstellt, beispielsweise bei Herzinfarktpatienten zur Verhütung eines plötzlichen Exitus («sudden death»), geeignet und können dementsprechend als Wirkstoff in antithrombotischen Arzneimitteln verwendet werden.

Die Erfindung betrifft in erster Linie Verbindungen der Formel I, worin R Carboxy, Niederalkoxycarbonyl, beispielsweise mit bis zu 5 C-Atomen, z.B. Methoxy- oder Äthoxycarbonyl, oder Phenylniederalkoxycarbonyl, beispielsweise mit bis zu 11 C-Atomen, z.B. Benzyloxycarbonyl oder 2-Phenyläthoxycarbonyl, bedeutet und Salze der Verbindung der Formel I, worin R Carboxy bedeutet, namentlich das 2-(2-Phenylthioäthyl)-malonsäure-N,N'-diphenyl-monohydrazid und seine Salze.

Die Verbindungen der Formel I können nach an sich bekannten Methoden hergestellt werden, beispielsweise indem man in einer Verbindung der Formel

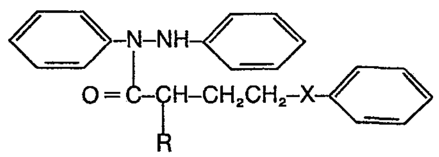

(II),

worin X einen reduktiv in die Thiogruppe überführbaren Rest bedeutet, wobei das sekundäre Stickstoffatom und/oder eine Carboxygruppe R in intermediär geschützter Form vorliegen kann, X zur Thiogruppe reduziert, erforderlichenfalls in der verfahrensgemäss erhältlichen Verbindung die Schutzgruppe(n) abspaltet, und, wenn erwünscht, die erhaltene Verbindung in eine andere Verbindung der Formel I überführt und/oder die gegebenenfalls erhaltene Verbindung der Formel I, worin R Carboxy bedeutet, in ein Salz oder ein erhaltenes Salz einer solchen in die freie Säure oder in ein anderes Salz überführt.

Der reduktiv in die Thiogruppe überführbare Rest X ist insbesondere die Sulfinylgruppe der Formel $-S(O)-$, ferner die Sulfonylgruppe der Formel $-S(O_2)-$.

Die Reduktion erfolgt in üblicher Weise, beispielsweise durch Behandlung mit einem aus der Literatur für analoge Reduktionen als geeignet bekannten selektiven Reduktionsmittel, vorteilhaft in einem organischen Lösungsmittel, erforderlichenfalls unter Kühlen oder Erwärmen, z.B. im Temperaturbereich von etwa 0 bis 100 °C, und/oder unter Inertgas, wie Stickstoff. Geeignete selektive Reduktionsmittel sind beispielsweise Leichtmetallhydride, wie Dichlorboran, beispielsweise in Tetrahydrofuran, Natriumborhydrid, beispielsweise in einem Niederalkanol, oder Phosphor-III-Verbindungen, z.B. Phosphortrichlorid oder Phosphorigsäure-brenzkatechin-phenylester, z.B. in Di-, Tri- oder Tetrachlormethan. Die Reduktion der Gruppe X kann aber auch durch Einwirkung von Wasserstoff in Gegenwart eines geeigneten Hydrierungskatalysators, wie Palladium auf Aluminiumoxid, vorteilhaft in einem Niederalkanol, erfolgen.

Die Ausgangsstoffe der Formel II sind zum Teil bekannt. Neue Verbindungen der Formel II können beispielsweise hergestellt werden, indem man eine Verbindung der Formel

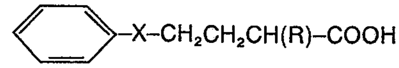

(IIa)

durch Behandlung mit Thionylchlorid in Benzol in das Monosäurechlorid überführt, dieses in Gegenwart von Diisopropyläthylamin und vorteilhaft in Toluol als Lösungsmittel mit N-Acetyl-N,N'-Diphenylhydrazin umsetzt, das erhaltene N-Acetylhydrazid durch Behandlung mit Natronlauge entacetyliert und aus dem erhaltenen Salz gewünschtenfalls die Säure freisetzt.

Die Verbindung der Formel I, worin R Carboxy bedeutet, kann man ferner herstellen, indem man in einer Verbindung der Formel III

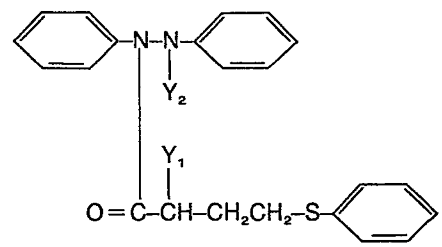

(III)

worin $Y_1$ eine funktionell abgewandelte Carboxygruppe und $Y_2$ Wasserstoff oder eine Amino-Schutzgruppe ist oder $Y_1$ und $Y_2$ gemeinsam Carbonyl darstellen, die funktionell abgewandelte Carboxygruppe $Y_1$ bzw. unter Spaltung der $Y_2$-N-Bindung die Carbonylgruppe ($Y_1 + Y_2$) solvolysiert, erforderlichenfalls die Schutzgruppe abspaltet, und gewünschtenfalls die erhaltene Säure in ein Salz oder ein erhaltenes Salz in die Säure oder in ein anderes Salz überführt.

Funktionell abgewandeltes Carboxy ist beispielsweise verestertes oder amidiertes Carboxy. Als veresterte Carboxygruppen kommen beispielsweise die eingangs als verestertes Carboxy R genannten oder von diesen verschiedene veresterte Carboxygruppen in Betracht. Von veresterten Carboxygruppen R verschiedene veresterte Carboxygruppen sind beispielsweise Phenoxycarbonyl, das gegebenenfalls, z.B. durch Niederalkyl, Niederalkoxy, Halogen und/oder Nitro, substituiert sein kann, Cycloalkyl- bzw. Cycloalkylniederalkoxycarbonyl, worin Cycloalkyl z.B. 5–7 Ringglieder aufweist, Tetrahydropyranyloxy- oder Benzhydryloxycarbonyl, oder durch Halogen oder Cyano substituiertes Niederalkoxycarbonyl, wie 2,2,2-Trichloräthoxy- oder Cyanomethoxycarbonyl. Als Amide der Formel III kommen beispielsweise offenkettige Amide, wie Amide mit Ammoniak oder primären oder sekundären organischen Aminen, wie Mono- oder Diniederalkylaminen, 5- bis 7gliedrigen Alkylen- bzw. 3-Aza-, 3-Oxa - oder 3-Thiaalkylenaminen, in Betracht.

Die Solvolyse erfolgt in üblicher Weise, vorzugsweise durch Hydrolyse in Gegenwart eines alkalischen Hydrolysemittels, vorteilhaft unter Erwärmen, z.B. im Temperaturbereich von etwa 50–150 °C, und erforderlichenfalls in Gegenwart eines mit Wasser mischbaren Lösungsmittels und/oder unter Inertgas, wie Stickstoff. Alkalische Hydrolysemittel sind beispielsweise anorganische Basen, wie Alkalimetallhydroxide oder -carbonate, z.B. Natrium- oder Kaliumhydroxid, Natrium- oder Kaliumcarbonat, oder Ammoniak, vorzugsweise in verdünnter, z.B. 0,5- bis 2normaler, Lösung, ferner mit Wasser mischbare organische Amine, z.B. Triäthylamin. Mit Wasser mischbare Lösungsmittel sind z.B. Niederalkanole, wie Methanol oder Äthanol, Dioxan oder Tetrahydrofuran.

Einige als Ausgangsstoffe zu verwendenden funktionellen Carboxyderivate der Säure der Formel I sind bekannt. Neue Ausgangsstoffe der genannten Art können nach an sich bekannten Methoden hergestellt werden. So kann man Ester derselben z.B. erhalten, indem man einen entsprechenden 2-(2-Phenylthioäthyl)-malonsäurehalbester in Gegenwart von N,N'-Dicyclohexylcarbodiimid in Tetrahydrofuran mit N,N'-Diphenylhydrazin kondensiert. Offenkettige Amide können in analoger Weise hergestellt werden, wobei man von entsprechenden 2-(2-Phenylthioäthyl)-malonsäurehalbamiden ausgeht.

Die Verbindungen der Formel I können ferner hergestellt werden, indem man Verbindungen der Formel IV und V

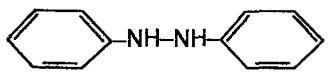

(IV),

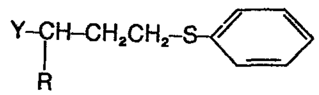

(V),

worin Y eine gegebenenfalls in Anhydridform vorliegende Carboxygruppe bedeutet, und wobei das nicht an der Reaktion teilnehmende Stickstoffatom der Komponente (IV) und/oder eine in der Komponente (V) gegebenenfalls vorhandene Carboxygruppe R in intermediär geschützter Form vorliegen kann, miteinander kondensiert, erforderlichenfalls in der verfahrensgemäss erhältlichen Verbindung die Schutzgruppe(n) entfernt und, wenn erwünscht, die erhaltene Verbindung in eine andere Verbindung der Formel I überführt, und/oder die gegebenenfalls erhaltene Verbindung der Formel I, worin R Carboxy bedeutet, in ein Salz oder ein erhaltenes Salz einer solchen in die freie Säure oder in ein anderes Salz überführt.

In Anhydridform vorliegendes Carboxy Y ist beispielsweise Halogencarbonyl, insbesondere Chlorcarbonyl, oder in zweiter Linie mit einer Niederalkancarbonsäure anhydriertes Carboxy, z.B. Formyloxycarbonyl, Acetoxycarbonyl oder Pivaloyloxycarbonyl.

Die Umsetzung erfolgt in an sich bekannter Weise, vorteilhaft in einem Lösungsmittel und erforderlichenfalls in Gegenwart eines Kondensationsmittels, unter Kühlen oder Erwärmen, z.B. im Temperaturbereich von etwa $-20$ bis 100 °C, insbesondere von etwa 0–50 °C, und/oder unter Inertgas, wie Stickstoff. Geeignete Lösungsmittel sind beispielsweise Di-, Tri- und Tetrachlormethan, 1,1,2,2-Tetrachloräthan, Dioxan, Tetrahydrofuran, Benzol oder Toluol. Als Kondensationsmittel kommen, ausgehend von Säureanhydriden beispielsweise basische Kondensationsmittel und ausgehend von Säuren der Formel V beispielsweise wasserbindende Mittel in Betracht. Basische Kondensationsmittel sind beispielsweise Alkalimetallniederalkanolate, wie Natriummethanolat, Alkalimetall- oder Erdalkalimetallhydroxide oder -carbonate, z.B. Natrium-, Kalium- oder Calciumhydroxid oder Natrium- oder Kaliumcarbonat, oder tertiäre organische Stickstoffbasen, wie Triniederalkylamine, z.B. Triäthylamin oder Tributylamin, tertiäre Anilinderivate, wie N-Diniederalkylaniline, z.B. N,N-Dimethylanilin, oder tertiäre heterocyclische Basen, wie Pyridin. Als wasserbindende Mittel sind insbesondere Carbodiimide, z.B. Dicyclohexylcarbodiimid, geeignet.

Die Ausgangsstoffe der Formel V sind zum Teil bekannt. Neue Ausgangsstoffe können beispielsweise ausgehend von der entsprechenden Säure der Formel V, worin Y Carboxy ist, hergestellt werden, beispielsweise durch Behandeln mit Thionylchlorid, vorzugsweise in Benzol bei etwa 50°, oder mit einem Niederalkansäureanhydrid oder -chlorid, wie dem gemischten Anhydrid von Ameisen- und Essigsäure, Acetanhydrid oder Acetylchlorid, in Gegenwart eines Alkalimetall- oder des Ammoniumsalzes der betreffenden Säure.

Die Verbindungen der Formel I können weiterhin hergestellt werden, indem man aus einer Verbindung der Formel VI

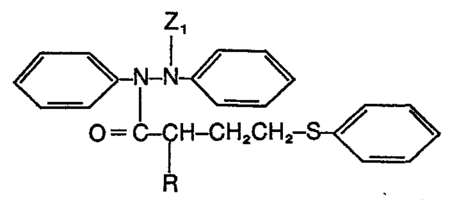

, (VI)

worin $Z_1$ einen unter Einführung von Wasserstoff abspaltbaren Rest bedeutet, und worin eine Carboxygruppe R in intermediär geschützter Form vorliegen kann, $Z_1$ unter Einführung von Wasserstoff abspaltet, erforderlichenfalls in der verfahrensgemäss erhältlichen Verbindung die Schutzgruppe entfernt, und, wenn erwünscht, die erhaltene Verbindung in eine andere Verbindung der Formel I umwandelt und/oder eine erhaltene Verbindung der Formel I, worin R Carboxy bedeutet, in ein Salz oder ein erhaltenes Salz einer solchen in die freie Säure oder in ein anderes Salz überführt.

Der abspaltbare Rest $Z_1$ ist beispielsweise Acyl, wie von einer Carbonsäure, z.B. einer organischen Carbonsäure oder einem Kohlensäurehalbester, abgeleitetes Acyl, gegebenenfalls substituiertes α-Phenylniederalkyl, Sulfonyl oder Silyl. Von einer organischen Carbonsäure abgeleitetes Acyl ist beispielsweise gegebenenfalls halogeniertes Niederalkanoyl, wie Acetyl, Trifluoracetyl, Propionyl oder Pivaloyl, oder gegebenenfalls, z.B. durch Niederalkyl, Niederalkoxy, Halogen und/oder Nitro, substituiertes Benzoyl. Von einem Kohlensäurehalbester abgeleitetes Acyl ist beispielsweise gegebenenfalls substituiertes, wie halogeniertes, Niederalkoxycarbonyl, wie Methoxy-, Äthoxy-, 2-Jodäthoxy-, 2,2,2-Tribromäthoxy-, 2,2,2-Trichloräthoxy- oder Tertiärbutyloxycarbonyl, 5- bis 7gliedriges Cycloalkoxycarbonyl, wie Cyclohexyloxycarbonyl, ge-gebenenfalls, z.B. durch Niederalkyl, Niederalkoxy, Halogen und/oder Nitro, substituiertes Phenylniederalkoxycarbonyl, vorzugsweise α-Phenylniederalkoxycarbonyl, z.B. Benzyloxycarbonyl. Gegebenenfalls substituiertes α-Phenylniederalkyl ist beispielsweise gegebenenfalls, z.B. durch Niederalkoxy, Halogen und/oder Nitro, substituiertes Benzyl. Sulfonylreste sind beispielsweise von organischen Sulfonsäuren abgeleitete Sulfonylreste, z.B. Methansulfonyl-, Äthansulfonyl- oder Benzol-, p-Toluol- oder p-Brombenzolsulfonyl. Silyl ist beispielsweise Triniederalkylsilyl, wie Trimethylsilyl.

Die Abspaltung des Restes $Z_1$ erfolgt beispielsweise durch Solvolyse oder Reduktion. So kann man Acylreste und Silylreste $Z_1$ hydrolytisch und α-Phenylniederalkyl-sowie α-Phenylniederalkoxycarbonylreste, halogenierte Niederalkoxycarbonylreste und Sulfonylreste reduktiv abspalten.

Die Solvolyse erfolgt in üblicher Weise, z.B. durch Hydrolyse in Gegenwart eines alkalischen Hydrolysemittels, vorteilhaft unter Erwärmen, z.B. im Temperaturbereich von etwa 50–150 °C, und erforderlichenfalls in Gegenwart eines mit Wasser mischbaren Lösungsmittels und/oder unter Inertgas, wie Stickstoff. Alkalischen Hydrolysemittel sind beispielsweise anorganische Basen, wie Alkalimetallhydroxide oder -carbonate, z.B. Natrium- oder Kaliumhydroxid, Natrium- oder Kaliumcarbonat, oder Ammoniak, vorzugsweise in verdünnter, z.B. 0,5- bis 2normaler Lösung, ferner mit Wasser mischbare organische Amine, z.B. Triäthylamin.

Die reduktive Abspaltung des Restes $Z_1$ erfolgt beispielsweise durch Einwirkung eines geeigneten Reduktionsmittels, vorteilhaft in einem unter den Reaktionsbedingungen inerten organischen Lösungsmittel, erforderlichenfalls unter Kühlen oder Erwärmen, z.B. im Temperaturbereich von etwa 0–150 °C, und/oder unter Inertgas, wie Stickstoff. Als Reduktionsmittel für die reduktive Abspaltung von α-Phenylniederalkyl und α-Phenylniederalkoxycarbonyl kommt beispielsweise Wasserstoff in Gegenwart eines Hydrierungs-Katalysators, wie eines Palladium- oder Rhodiumkatalysators, z.B. von Palladium oder Rhodium auf Aluminiumoxid, in Betracht. Bei dieser Verfahrensvariante kann es besonders vorteilhaft sein, von einer Verbindung der Formel VII auszugehen, worin R α-Phenylalkoxycarbonyl darstellt. Da dies ebenfalls, und zwar zu Carboxy, reduziert wird, gelangt man so leicht zu der Säure der Formel I. Halogenierte Niederalkoxycarbonylreste und Sulfonylreste können beispielsweise durch Behandeln mit Zink in Gegenwart von verdünnter Essigsäure, halogenierte Niederalkoxycarbonylreste auch durch Umsetzung mit Chrom-II-chlorid oder Chrom-II-acetat, α-Phenylniederalkoxycarbonyl auch durch Behandlung mit Natrium in verflüssigtem Ammoniak, abgespalten werden.

Die Ausgangsstoffe der Formel VI können beispielsweise hergestellt werden, indem man eine Verbindung der Formel

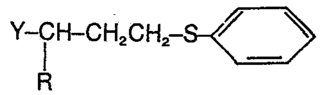 (V),

worin Y gegebenenfalls in Anhydridform vorliegendes Carboxy bedeutet, mit einem N,N′-Diphenyl-N-$Z_1$-hydrazin umsetzt.

Die Umsetzung erfolgt in an sich bekannter Weise, vorteilhaft in einem Lösungsmittel und erforderlichenfalls in Gegenwart eines Kondensationsmittels, unter Kühlen oder Erwärmen, z.B. im Temperaturbereich von etwa −20–100 °C, insbesondere von etwa 0–50 °C, und/oder unter Inertgas, wie Stickstoff. Geeignete Lösungsmittel sind beispielsweise Di-, Tri- und Tetrachlormethan, 1,1,2,2-Tetrachloräthan, Dioxan, Tetrahydrofuran, Benzol oder Toluol. Als Kondensationsmittel kommen beispielsweise basische Kondensationsmittel, ausgehend von Säuren der Formel V, beispielsweise ferner wasserbindende Mittel in Betracht. Basische Kondensationsmittel sind beispielsweise Alkalimetallniederalkanolate, wie Natriummethanolat, Alkalimetall- oder Erdalkalimetallhydroxide oder -carbonate, z.B. Natrium-, Kalium- oder Calciumhydroxid oder Natrium- oder Kaliumcarbonat, oder tertiäre organische Stickstoffbasen, wie Triniederalkylamine, z.B. Diisopropylamin oder Tributylamin, tertiäre Anilinderivate, wie N,N-Diniederalkylaniline, z.B. N,N-Dimethylanilin, oder tertiäre heterocyclische Basen, wie Pyridin. Als wasserbindende Mittel sind insbesondere Carbodiimide, z.B. Dicyclohexylcarbodiimid, geeignet.

Die als Ausgangsstoffe für diese Umsetzung dienenden N,N′-Diphenyl-N-$Z_1$-hydrazine können z.B. durch Umsetzung von N,N-Diphenylhydrazin mit einem Carbonsäureanhydrid, z.B. Acetanhydrid, Acetylchlorid oder Trifluoracetanhydrid, Chlor- oder Bromameisensäureester, z.B. Chlorameisensäurebenzylester, Phenylniederalkylhalogenid, z.B. Benzylbromid, Sulfonsäureanhydrid, z.B. p-Toluolsulfonchlorid, oder einem Triniederalkylchlorsilan, z.B. Trimethylchlorsilan, erhalten werden.

Die Verbindungen der Formel I können ferner hergestellt werden, indem man in einer Verbindung der Formel VII

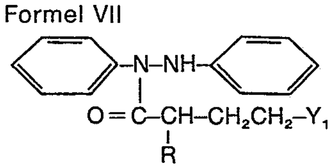 (VII)

worin $Y_1$ einen durch die Phenylthiogruppe ersetzbaren Rest bedeutet, und worin das sekundäre Stickstoffatom und/oder eine Carboxygruppe R in intermediär geschützter Form vorliegen kann, $Y_1$ durch Phenylthio ersetzt, erforderlichenfalls in der verfahrensgemäss erhältlichen Verbindung die Schutzgruppe(n) entfernt, und, wenn erwünscht, die erhaltene Verbindung in eine andere Verbindung der Formel I, worin R Carboxy bedeutet, in ein Salz oder ein erhaltenes Salz einer solchen in die freie Säure oder in ein anderes Salz überführt.

Der durch die Phenylthiogruppe ersetzbare Rest $Y_1$ ist beispielsweise eine reaktionsfähige veresterte Hydroxygruppe, wie mit einer Halogenwasserstoffsäure oder einer organischen Sulfonsäure verestertes Hydroxy, z.B. Chlor, Brom, Methansulfonyloxy, Äthansulfonyloxy oder Benzol-, Toluol- oder p-Brombenzolsulfonyloxy.

Der Ersatz von $Y_1$ durch Phenylthio kann in üblicher Weise erfolgen, beispielsweise durch Behandeln mit Thiophenol oder einem Salz, wie einem Alkalimetallsalz, davon, z.B. mit Natriumthiophenolat, vorteilhaft in einem unter den Reaktionsbedingungen inerten Lösungsmittel, z.B. einem Äther, wie Diäthyläther, Dioxan oder Tetrahydrofuran, Benzol, Toluol oder einem Niederalkanol, wie Methanol, erforderlichenfalls unter Kühlen oder Erwärmen, z.B. im Temperaturbereich von etwa 0–100 °C, und/oder unter Inertgas, wie Stickstoff.

Die Ausgangsstoffe der Formel VII können beispielsweise hergestellt werden, indem man eine 2-(2-$Y_1$-Äthyl)-malonsäure bzw. einen Halbester oder das Halbchlorid davon in Gegenwart von Dicyclohexylcarbodiimid mit N,N'-Diphenylhydrazin behandelt.

Wie erwähnt, können an den vorstehend beschriebenen Umsetzungen nicht teilnehmende funktionelle Gruppen, das sind Carboxygruppen R in Ausgangsstoffen der Formel II, V, VI und VII, das sekundäre Anilinstickstoffatom in Ausgangsstoffen der Formeln II, III und VII und eines der Stickstoffatome in Ausgangsstoffen der Formel IV, in intermediär geschützter Form vorliegen und während der oder anschliessend an die eigentliche Reaktion wieder freigesetzt werden. Als Schutzgruppen sind die aus der chemischen Literatur bekannten Carboxy- bzw. Aminoschutzgruppen geeignet. Als Carboxyschutzgruppen sind vor allem esterartig gebundene Carboxyschutzgruppen zu nennen, die im neutralen oder alkalischen Milieu abgespaltet werden können, und die verestertes Carboxy R oder Phenoxycarbonyl, das gegebenenfalls, z.B. Durch Niederalkyl, Niederalkoxy, Halogen und/oder Nitro, substituiert sein kann, Cycloalkyl- bzw. Cycloalkylniederalkoxycarbonyl, worin Cycloalkyl z.B. 5–7 Ringglieder aufweist, Tetrahydropyranyloxy- oder Benzhydryloxycarbonyl oder durch Halogen oder Cyano substituiertes Niederalkyloxycarbonyl, wie 2,2,2-Trichloräthoxy- oder Cyanomethyloxycarbonyl bilden. Als Amin-Schutzgruppen sind vor allem neutral oder basisch abspaltbare Amin-Schutzgruppen, beispielsweise Acyl, wie von einer Carbonsäure oder einem Kohlensäurehalbester, abgeleitetes Acyl, ein gegebenenfalls substituiertes α-Phenylniederalkyl, Sulfonyl oder Silyl zu nennen. Ein von einer organischen Carbonsäure abgeleiteter Acylrest ist beispielsweise gegebenenfalls halogeniertes Niederalkanoyl, wie Acetyl, Trifluoracetyl, Propionyl oder Pivaloyl, oder gegebenenfalls, z.B. durch Niederalkyl, Niederalkoxy, Halogen und/oder Nitro substituiertes Benzoyl. Ein von einem Kohlensäurehalbester abgeleiteter Acylrest ist beispielsweise gegebenenfalls substituiertes, wie halogeniertes, Niederalkoxycarbonyl, wie Methoxy-, Äthoxy-, 2-Jodäthoxy-, 2,2,2-Tribromäthoxy-, 2,2,2-Trichloräthoxy- oder Tertiärbutyloxycarbonyl, 5- bis 7gliedriges Cycloalkoxycarbonyl, wie Cyclohexyloxycarbonyl, gegebenenfalls, z.B. durch Niederalkyl, Niederalkoxy, Halogen und/oder Nitro, substituiertes Phenylniederalkoxycarbonyl, vorzugsweise α-Phenylniederalkoxycarbonyl, z.B. Benzyloxycarbonyl. Ein gegebenenfalls substituierter α-Phenylniederalkoxycarbonyl ist beispielsweise gegebenenfalls z.B. durch Niederalkyl, Niederalkoxy, Halogen und/oder Nitro, substituiertes Benzyl. Sulfonylreste sind beispielsweise von organischen Sulfonsäuren abgeleitete Sulfonylreste, z.B. Methansulfonyl-, Äthansulfonyl- oder Benzol-, p-Toluol- oder p-Brombenzolsulfonyl. Ein Silylrest ist beispielsweise Triniederalkylsilyl, wie Trimethylsilyl. Für die Abspaltung der genannten Schutzgruppen kommen die für die Entfernung solcher und analoger Schutzgruppen bekannter Methoden, beispielsweise die vorstehend erwähnten, in Frage.

Eine verfahrensgemäss erhältliche Verbindung kann man in eine andere Verbindung der Formel I überführen.

So kann man beispielsweise die erfindungsgemäss erhältliche Säure der Formel I, worin R Carboxy ist, nach üblichen neutralen oder basischen Veresterungsverfahren verestern.

So kann man z.B. durch Behandeln mit einer geeigneten Diazoverbindung, wie einem Diazoniederalkan, mit einem geeigneten N,N-Diniederalkylformamidacetal, z.B. N,N-Dimethylformamiddiäthylacetal oder N,N,N-Trimethylformamid-methosulfat, oder einem Oxoniumsalz, wie mit einem Triniederalkyloxonium-tetrafluoroborat oder -hexyfluorophosphat, mit einem Carbonat oder Pyrocarbonat, z.B. mit Diäthyl(pyro)carbonat, oder mit einem Alkohol in Gegenwart eines geeigneten Kondensationsmittels, wie eines dehydratisierenden Mittels, z.B. Dicyclohexylcarbodiimid, oder, zur Bildung einer Hydroxyniederalkylgruppe, mit einem Epoxyniederalkan, z.B. Äthylenoxyd, verestern. Ferner kann man eine Verbindung der Formel I, worin eine freie Carboxylgruppe R in Salzform, z.B. in der Alkalimetall- wie Natriumsalzform, vorliegt, mit einem reaktionsfähigen Ester eines Alkohols, z.B. mit einer starken Säure, wie einem entsprechenden Halogenid, z.B. Chlorid, Bromid oder Jodid, oder Schwefelsäureester umsetzen, oder eine Verbindung der Formel I, worin R eine Carboxylgruppe ist, am sekundären Stickstoffatom intermediär schützen; dann durch Behandeln mit einem Halogenierungsmittel, z.B. Thionylchlorid, in Halogencarbonyl überführen, anschliessend mit einem Methallalkoholat oder einem Alkohol in Gegenwart einer säurebindenden Base umsetzen, die Schutzgruppe abspalten, und so zu einer Verbindung der Formel I gelangen, worin R für verestertes Carboxy steht. Dabei können in einem Veresterungsreagens gegebenenfalls vorhandene Substituenten in funktionell abgewandelter Form vorliegen und dann in einer Verbindung der Formel I, worin R z.B. für substituiertes Niederalkoxycarbonyl steht, in welchem Substituenten in funktionelle abgewandelter Form vorliegen, freigesetzt werden. So kann man als Veresterungsreagens z.B. das 2,3-Epoxy-propylchlorid verwenden und im erhaltenen Ester eine 2,3-Epoxy-propyloxygruppe nachträglich zur gewünschten 2,3-Dihydroxypropyloxy-Gruppierung hydrolysieren.

In einer Verbindung der Formel I, worin R für verestertes Carboxy, z.B. auch p-Nitro- bzw. 2,4-Dinitrophenoxy- oder -benzyloxycarbonyl steht, kann dieses durch Umesterung, z.B. durch Behandeln mit einem Alkohol, erforderlichenfalls in Gegenwart eines geeigneten Umesterungskatalysators, wie eines gegebenenfalls substituierten Alkalimetalls-, z.B. Natrium- oder Kaliumalkonolats, in eine andere veresterte Carboxygruppe umgewandelt werden.

In einer Verbindung der Formel I, worin R für verestertes Carboxy steht, kann dieses in an sich bekannter Weise in Carboxy überführen, beispielsweise durch Hydrolyse oder ausgehend von in Phenylteil gegebenenfalls substituierten α-Phenylniederalkylestern durch Reduktion.

Die Hydrolyse erfolgt in üblicher Weise, vorzugsweise in Gegenwart eines alkalischen Hydrolysemittels, vorteilhaft unter Erwärmen, z.B. im Temperaturbereich von etwa 50–150 °C, und erforderlichenfalls in Gegenwart eines mit Wasser mischbaren Lösungsmittels und/oder unter Inertgas, wie Stickstoff. Alkalische Hydrolysemittel sind beispielsweise anorganische Basen, wie Alkalimetallhydroxide oder -carbonate, z.B. Natrium- oder Kaliumhydroxid, Natrium- oder Kaliumcarbonat, oder Ammoniak, vorzugsweise in verdünnter, z.B. 0,5- bis 2normaler Lösung, ferner mit Wasser mischbare organische Amine, z.B. Triäthylamin.

Die Reduktion erfolgt vorzugsweise durch Einwirkung von Wasserstoff in Gegenwart eines Hydrierungskatalysators. Als Hydrierungskatalysatoren kommen beispielsweise Edelmetallkatalysatoren, wie Palladium- oder Rhodiumkatalysatoren, z.B. von Palladium oder Rhodium auf Aluminiumoxid, in Betracht. Vorteilhaft arbeitet man in Gegenwart einer Base, wie einer tertiären Stickstoffbase, z.B. von Triäthylamin, und in einem unter den Reduktionsbedingungen inerten organischen Lösungsmittel, z.B. Dioxan, erforderlichenfalls unter Überdruck und/oder unter gelindem Erwärmen, z.B. im Druckbereich von etwa 1 bis 5 bar und im Temperaturbereich von etwa 0–5 °C. Die Reduktion kann aber auch durch Behandeln mit Natrium in verflüssigtem Ammoniak bewirkt werden.

Erhaltene freie Verbindungen der Formel I können in an sich bekannter Weise in Salze überführt werden, z.B. durch Behandeln mit der entsprechenden Base, üblicherweise in Gegenwart eines Lösungs- oder Verdünnungsmittels.

Erhaltene Salze können in an sich bekannter Weise in die freien Verbindungen umgewandelt werden, z.B. durch Behandeln mit einer Säure, wie einem Äquivalent einer Mineralsäure.

Die Verbindungen der Formel I, einschliesslich ihrer Salze, können auch in Form ihrer Hydrate erhalten werden oder das zur Kristallisation verwendete Lösungsmittel einschliessen.

Infolge der engen Beziehung zwischen der neuen Verbindung der Formel I, worin R Carboxy ist, in freier Form ind in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt, oder einen Ausgangsstoff in Form eines Salzes und/oder Racemates bzw. Antipoden verwendet oder insbesondere unter den Reaktionsbedingungen bildet.

Bei den erfindungsgemässen pharmazeutischen Präparaten handelt es sich um solche, die zur enteralen, wie oralen oder rektalen, ferner parenteralen Verabreichung an Warmblüter bestimmt sind und den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Die neuen pharmazeutischen Präparate enthalten z.B. von etwa 10 bis 95%, vorzugsweise von etwa 20 bis 90% des Wirkstoffs. Erfindungsgemäss pharmazeutische Präparate sind z.B. solche in Dosiseinheitsformen, wie Dragées, Tabletten, Kapseln oder Suppositorien, ferner Ampullen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt.

So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet. Geeignete Trägerstoffe sind insbesondere Füllstoffe, wie Zucker, z.B. Lactose, Saccarose, Mannit oder Sorbit, Cellulosepräparate und/oder Calciumphosphate, z.B. Tricalciumphosphat oder Calciumhydrogenphosphat, ferner Bindemittel, wie Stärkekleister, z.B. von Mais-, Weizen-, Reis- oder Kartoffelstärkekleister, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi,

Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei gegebenenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Äthyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Die Erfindung betrifft ebenfalls die Verwendung der neuen Verbindungen der Formel I und ihrer Salze als Arzneimittel, insbesondere als Antithrombotika, vorzugsweise in der Form von pharmazeutischen Präparaten. Die für einen etwa 75 kg schweren Wärmblüter empfohlene Tagesdosis beträgt etwa 0,1–1 g, vorzugsweise 0,25–0,75 g, vorteilhaft auf 3 oder 4 gleiche Tagesdosen verteilt.

Die nachfolgenden Beispiele illustrieren die oben beschriebene Erfindung; sie sollen diese jedoch in ihrem Umfang in keiner Weise einschränken. Temperaturen werden in Celsiusgraden angegeben.

Beispiel 1

Eine Lösung von 3,88 g 1,2-Diphenyl-4-(2-phenylthioäthyl)-pyrazolidin-3,5-dion in 10,25 ml n- Natrolauge wird 23 Stunden unter Stickstoff bei einer Badtemperatur von 140° zum Rückfluss erhitzt. Man gibt 5 ml Wasser zum Reaktionsgemisch hinzu und erhitzt weitere 23 Stunden zum Rückfluss. Dann wird die Suspension auf Raumtemperatur abgekühlt und filtriert. Das Filtrat wird bis zum Umschlag von Kongoblau mit 2n Salzsäure versetzt und dann zweimal mit je 50 ml Essigester extrahiert. Die vereinigten organischen Phasen werden zweimal mit je 20 ml Wasser gewaschen, über Magnesiumsulfat getrocknet und bei einer Badtemperatur von 40° unter vermindertem Druck eingedampft. Der Rückstand, ein gelbes Öl, wird in 50 ml Essigester gelöst, die Lösung zweimal mit je 25 ml 2n Kaliumhydrogencarbonatlösung und anschliessend zweimal mit je 50 ml 0,5n Natriumcarbonatlösung extrahiert. Die Natriumcarbonatlösungen werden vereinigt und bei 5° mit konzentrierter Salzsäure angesäuert. Das ausgeschiedene Öl wird zweimal mit je 50 ml Essigester extrahiert. Die vereinigten organischen Phasen werden mit 20 ml Wasser gewaschen, über Magnesiumsulfat getrocknet und bei einer Badtemperatur von 40° unter vermindertem Druck zur Trockne eingedampft. Der Rückstand, ein Öl, wird an 500 g Silicagel chromatographiert. Die Fraktionen 1–14, eluiert mit je 200 ml Toluol, sowie die Fraktionen 15–18, eluiert mit je 200 ml Toluol-Essigsäureäthylester, werden verworfen. Die Fraktionen 19 und 20, eluiert mit je 200 ml Toluol-Essigsäureäthylester, werden vereinigt und im Vakuum eingedampft. Der Rückstand wird in 50 ml Aceton gelöst. Die ätherische Lösung wird bis zur Trübung mit Petroläther versetzt. Nach einigem Stehen kristallisiert das 2-(2-Phenylthioäthyl)-malonsäure-monoäthylester-N,N'-diphenylhydrazid als farblose Kristalle aus. Smp. 82–84°.

Beispiel 2

Zu einer Lösung von 22,9 g 2-(2-Phenylthioäthyl)-malonsäure-monoäthylester und 15,7 g frisch umkristallisiertem Hydrazobenzol in 160 ml absolutem Tetrahydrofuran lässt man unter Stickstoff bei 5–10° eine Lösung von 17,9 g Dicyclohexylcarbodiimid in 95 ml absolutem Tetrahydrofuran zutropfen. Hierauf wird die Mischung 15 Stunden bei 25° unter Stickstoff gerührt, dann der ausgeschiedene Dicyclohexylharnstoff abfiltriert und das Filtrat nach Zusatz von 3 Tropfen Eisessig im Vakuum zur Trockne eingedampft. Der Rückstand wird in 1 Liter Äther gelöst, die ätherische Lösung zweimal mit je 100 ml 2n Kaliumhydrogencarbonatlösung und zweimal mit je 100 ml 2n Salzsäure extrahiert und anschliessend mit Wasser neutral gewaschen. Hierauf wird die ätherische Lösung über Natriumsulfat getrocknet und im Vakuum eingeengt. Der Rückstand, ein Öl, wird an 500 g Silicagel chromatographiert. Die Fraktionen 1–14, eluiert mit je 200 ml Toluol, sowie die Fraktionen 15–18, eluiert mit je 200 ml Toluol-Essigsäureäthylester, werden verworfen. Die Fraktionen 19 und 20, eluiert mit je 200 ml

Toluol-Essigsäureäthylester, werden vereinigt und im Vakuum eingedampft. Der Rückstand wird in 50 ml Äther gelöst. Die ätherische Lösung wird bis zur Trübung mit Petroläther versetzt. Nach einigem Stehen kristallisiert das 2-(2-Phenylthioäthyl)-malonsäure-monoäthylester-N,N'-diphenyl hydrazid als farblose Kristalle aus. Smp. 82–84°.

Beispiel 3

Man erhitzt eine Suspension von 8,9 g 2-(2-Phenylthioäthyl)-malonsäure-monoäthylester-N,N'-diphenylhydrazid in 40 ml wässriger 0,5n Natronlauge 12 Stunden unter Stickstoff zum Rückfluss. Die Lösung wird filtriert und durch Extraktion mit Äther von Spuren Hydrazobenzol befreit. Die wässrige Lösung wird bei 10° mit konzentrierter Salzsäure kongosauer gestellt, das ausgeschiedene Öl in 40 ml Äthylacetat gelöst, die Lösung abgetrennt und mit Wasser neutral gewaschen. Hierauf wird die Lösung zweimal mit je 36 ml 0,5n Natriumcarbonatlösung ausgezogen. Die Auszüge werden getrennt mit konzentrierter Salzsäure kongosauer gestellt, mit Äthylacetat extrahiert, die Extrakte mit wenig Wasser neutral gewaschen, getrocknet und unter Vakuum eingedampft. Das zurückbleibende farblose Öl wird zweimal aus Essigsäureäthylester umkristallisiert, wobei man 2-(2-Phenylthioäthyl)-malonsäure-N,N'-diphenyl-monohydrazid erhält, Smp. 158–160°.

Beispiel 4

0,5 g 2-(2-Phenylthioäthyl)-malonsäure-monobenzylester-N,N'-diphenylhydrazid werden in 40 ml Methanol gelöst und nach Zusatz von 0,4 g Palladium auf Aluminiumoxid (5%) bei Raumtemperatur und Normaldruck hydriert. Nach 12 Stunden wird nochmals 0,2 g Katalysator zugesetzt und nach insgesamt 24 Stunden wird die Hydrierung abgebrochen. Der Katalysator wird abfiltriert und das Filtrat unter vermindertem Druck zur Trockne eingedampft. Den Rückstand löst man in 15 ml Essigsäureäthylester. Die organische Phase wird bei 0° zweimal mit je 10 ml 2n Natriumcarbonatlösung extrahiert. Die vereinigten wässrig-alkalischen Lösungen werden mit 2n Salzsäure angesäuert. Das ausgeschiedene Öl wird mit 20 ml Chloroform extrahiert und der Chloroformextrakt über Magnesiumsulfat getrocknet und unter vermindertem Druck eingedampft. Den Rückstand chromatographiert man an 20 g Silicagel. Die Fraktionen 1–7, eluiert mit je 50 ml Chloroform-Methanol (9:1), werden verworfen. Die Fraktionen 8–10, eluiert mit demselben Gemisch, werden vereinigt und unter vermindertem Druck zur Trockne eingedampft. Den Rückstand kristallisiert man aus Essigsäureäthylester. Das 2-(2-Phenylthioäthyl)-malonsäure-N,N'-diphenylhydrazid schmilzt bei 158–160 °C.

Das Ausgangsmaterial kann z. B. folgendermassen hergestellt werden:

Eine Lösung von 28,4 g Malonsäuredibenzylester in 150 ml Dimethylformamid wird unter Rühren und Feuchtigkeitsausschluss während 45 Minuten bei 0° portionsweise mit 4,8 g einer Natriumhydrid-Mineralöldispersion (50%) versetzt. Die Mischung wird 2 Stunden bei Raumtemperatur gerührt und anschliessend tropfenweise mit einer Lösung von 2-Phenylthioäthylbromid in 50 ml Dimethylformamid versetzt. Anschliessend rührt man 20 Stunden bei 50°, kühlt ab und giesst die Mischung auf 1,5 l Eiswasser. Das ausgeschiedene Öl wird durch zweimalige Extraktion mit je 500 ml Äther gelöst. Die vereinigten Ätherlösungen werden mit 200 ml Wasser gewaschen, über Magnesiumsulfat getrocknet und unter vermindertem Druck eingedampft. Der Rückstand wird an 1000 g Silicagel chromatographiert. Die Fraktionen 1–4, eluiert mit je 500 ml Toluol, werden verworfen. Die Fraktionen 5–25 enthalten den 2-(2-Phenylthioäthyl)-malonsäuredibenzylester. Die Fraktionen werden vereinigt und unter vermindertem Druck zur Trockne eingedampft. Der Ester liegt als farbloses Öl vor.

21,2 g 2-(2-Phenylthioäthyl)-malonsäuredibenzylester werden in 70 ml Dioxan gelöst. Die Lösung wird auf 70° erhitzt und unter Rühren mit 50,5 ml n Natriumhydroxidlösung versetzt. Man rührt 20 Minuten bei 70°, kühlt ab und destilliert das Dioxan bei 40° unter vermindertem Druck ab. Die wässrige Lösung wird mit 100 ml Äther überschichtet und bei 0° mit 35 ml 2n Salzsäure angesäuert. Die Ätherphase wird abgetrennt und die wässrige Phase zweimal mit je 100 ml Äther extrahiert. Die vereinigten Ätherphasen werden mit 100 ml 0,5n Kaliumbicarbonatlösung und anschliessend dreimal mit je 50 ml 2n Natriumcarbonatlösung extrahiert. Die vereinigten Natriumcarbonatlösungen werden bei 0° mit 2n Salzsäure angesäuert. Das ausgeschiedene Öl wird durch dreimalige Extraktion mit je 60 ml Chloroform gelöst. Die vereinigten Chloroformlösungen werden über Magnesiumsulfat getrocknet und unter vermindertem Druck eingedampft. Den Rückstand trocknet man 15 Stunden unter 0,13 mbar bei Raumtemperatur. Der 2-(2-Phenylthioäthyl)-malonsäure-monobenzylester liegt als farbloses Öl vor.

Eine Lösung von 4,8 g 2-(2-Phenylthioäthyl)-malonsäure-monobenzylester in 30 ml wasserfreiem Tetrahydrofuran wird unter Rühren und Einleiten von Stickstoff auf 0° abgekühlt. Man setzt 2,7 g Hydrazobenzol und anschliessend tropfenweise eine Lösung von 3,05 g Dicyclohexylcarbodiimid in 15 ml wasserfreiem Tetrahydrofuran zu. Die Mischung wird 15 Stunden bei Raumtemperatur gerührt und abgenutscht. Das Filtrat wird mit 3 Tropfen Eisessig versetzt und unter vermindertem Druck zur Trockne eingedampft. Den Rückstand löst man in 150 ml Äther. Die Ätherphase wird zweimal mit je 25 ml 2n Salzsäure, zweimal mit je 25 ml 2n Natriumhydrogencarbonatlösung und dann mit 30 ml Wasser extrahiert, über Magnesiumsulfat getrocknet und unter vermindertem Druck zur Trockne eingedampft. Der Rückstand wird an 120 g Silicagel chromatographiert. Die Fraktionen 1–4, eluiert mit je 200 ml Toluol sowie die Fraktionen 5 und 6, eluiert mit je 100 ml Toluol/Essigsäureäthylester (9:1), werden verworfen. Die Fraktionen 7–9, eluiert mit je 100 ml Toluol/Essigsäureäthylester (9:1), werden vereinigt und

unter vermindertem Druck zur Trockne eingedampft. Der Rückstand wird aus Diäthyläther/Petroläther kristallisiert. Man erhält das 2-(2-Phenylthioäthyl)-malonsäure-monobenzylester-N,N'-diphenylhydrazid vom Smp. 88–89°.

Beispiel 5

Eine Lösung von 4,76 g 2-(2-Phenylthioäthyl)-malonsäure-monoäthylester-N'-acetyl-N,N'-diphenylhydrazid (Smp. 76–78°, DOS 2 023 415) in 100 ml Äthanol und 10,0 ml n Natronlauge wird 3 Stunden bei Raumtemperatur gerührt und dann das Äthanol unter vermindertem Druck bei 40° abdestilliert. Der wässrige Rückstand wird mit 50 ml Wasser verdünnt und anschliessend zweimal mit je 40 ml Methylenchlorid extrahiert. Die Methylenchloridlösung, welche das Natriumsalz der gesuchten Verbindung enthält, wird abgetrennt und zweimal mit je 20 ml 2n Natriumcarbonat extrahiert. Die Natriumcarbonatlösungen werden vereinigt und mit 2n Salzsäure bei 5° sauer gestellt. Das ausgeschiedene Öl wird in 60 ml Methylenchlorid gelöst, die Methylenchloridlösung über Magnesiumsulfat getrocknet und bei einer Badtemperatur von 40° zur Trockne eingedampft. Den Rückstand kristallisiert man aus Äthanol. Das 2-(2-Phenylthioäthyl)-malonsäure-N'-acetyl-N,N'-diphenylhydrazid schmilzt bei 159–161°.

Beispiel 6

Eine Lösung von 1,8 g 2-(2-Phenylthioäthyl)-malonsäure-N'-acetyl-N,N'-diphenylhydrazid in 22 ml 0,5n Natronlauge wird 15 Stunden bei 60° unter Rühren erhitzt. Man kühlt ab und löst das ausgeschiedene Öl durch Zusatz von 20 ml Wasser. Die wässrige Phase wird dreimal mit je 20 ml Toluol extrahiert und anschliessend mit 2n Salzsäure auf pH 1 angesäuert. Das ausgeschiedene Öl wird dreimal mit je 60 ml Chloroform extrahiert. Die vereinigten Chloroformlösungen werden über Magnesiumsulfat getrocknet, abfiltriert und unter vermindertem Druck bei 40° zur Trockne eingedampft. Nach Kristallisation aus Äthylacetat-Hexan schmilzt das 2-(2-Phenylthioäthyl)-malonsäure-N,N'-diphenylhydrazid bei 158–160°.

Beispiel 7

0,4 g 2-(2-Phenylthioäthyl)-malonsäure-N,N'-diphenyl-monohydrazid werden in 1,0 ml n Natronlauge gelöst. Die Lösung wird mit 3 ml Wasser verdünnt und unter Rühren mit 73,5 mg Calciumchlorid-dihydrat zugesetzt. Die ausgeschiedenen Kristalle werden abfiltriert und mit 4,0 ml Wasser nachgewaschen. Das Calciumsalz schmilzt bei 143–147°.

Beispiel 8

Eine Lösung von 3,0 g 2-(2-Phenylthioäthyl)-malonsäure-monoäthylester-N'-acetyl-N,N'-diphenylhydrazid (Smp. 76–78°) in 20 ml Äthanol und 15 ml n Natronlauge wird 2 Stunden zum Rückfluss erhitzt und anschliessend der Alkohol unter vermindertem Druck bei 50° abdestilliert. Man versetzt mit 20 ml Wasser, kühlt auf 10° ab, filtriert, säuert das Filtrat bei 5° mit 2n Salzsäure an und extrahiert das ausgeschiedene Öl zweimal mit je 40 ml Äthylacetat. Die vereinigten organischen Phasen werden zweimal mit je 10 ml Wasser gewaschen, über Magnesiumsulfat getrocknet und bei einer Badtemperatur von 40° unter vermindertem Druck zur Trockne eingedampft. Der Rückstand, das 2-(2-Phenylthioäthyl)-malonsäure-N,N'-diphenyl-monohydrazid, schmilzt bei 158–160°.

Beispiel 9

Lacktabletten enthaltend 0,3 g 2-(2-Phenylthioäthyl)-malonsäure-N,N'-diphenyl-monohydrazid können wie folgt hergestellt werden:

Zusammensetzung (für eine Tablette),

| | |
|---|---|
| 2-(2-Phenylthioäthyl)-malonsäure-N,N'-diphenyl-monohydrazid | 0,300 g |
| Maisstärke | 0,088 g |
| Kolloidale Kieselsäure | 0,020 g |
| Magnesiumstearat | 0,002 g |
| Stearinsäure | 0,005 g |
| Natriumcarboxymethylstärke | 0,025 g |
| Wasser | q.s. |

Die Lacktabletten werden wie folgt hergestellt (für 10 000 Tabletten):

Das Gemisch von 3,0 kg 2-(2-Phenylthioäthyl)-malonsäure-N,N'-diphenyl-monohydrazid und 200 g kolloidaler Kieselsäure wird mit einem Stärkekleister aus 450 g Maisstärke und 2,2 kg entmineralisiertem Wasser zu einer feuchten Masse verarbeitet. Diese wird durch ein Sieb von 3 mm Maschenweite getrieben und bei 45° während 30 Minuten im Wirbelschichttrockner getrocknet. Das trockene Granulat wird durch ein Sieb von 1 mm Maschenweite gedrückt, mit einer zum voraus gesiebten Mischung (1 mm Sieb) von 130 g Maisstärke, 20 g Magnesiumstearat, 50 g Stearinsäure und 250 g Natriumcarboxymethylstärke gemischt und zu schwach gewölbten Tabletten von 6 mm Durchmesser (mit Bruchkerbe) gepresst.

Die nach dem obigen Verfahren herstellbaren Tabletten werden wie folgt mit einer Lackschicht versehen:

Die Presslinge werden in einem Dragierkessel von 45 mm Durchmesser mit einer Lösung von 20 g Schellack und 40 g Hydroxypropylmethylcellulose (niedere Viskosität) in 110 g Methanol und 1350 g Methylenchlorid durch gleichmässiges Aufsprühen während 30 Minuten überzogen; dabei wird durch gleichzeitiges Einblasen von Luft bei 60° getrocknet.

Anstelle des 2-(2-Phenylthioäthyl)-malonsäure-N,N'-diphenyl-monohydrazides kann auch die entsprechende Menge eines ihrer Salze, z.B. des Natriumsalzes, verwendet werden.

Beispiel 10

Tabletten, enthaltend 0,25 g 2-(2-Phenylthioäthyl)-malonsäure-N,N'-diphenyl-monohydrazid oder die entsprechende Menge ihres Natriumsalzes können z.B. wie folgt hergestellt werden:

Zusammensetzung (für 1 Tablette)

| | |
|---|---|
| 2-(2-Phenylthioäthyl)-malonsäure-N,N'-diphenyl-monohydrazid | 250 mg |
| Lactose | 100 mg |
| Weizenstärke | 200 mg |
| Kolloidale Kieselsäure | 24 mg |
| Magnesiumstearat | 4 mg |
| Talk | 22 mg |
| Wasser | q.s. |

Die Tabletten werden wie folgt hergestellt:

Die Wirkstoffe werden mit einem Teil der Weizenstärke, mit der Lactose und der kolloidalen Kieselsäure vermischt und das Gemisch durch ein Sieb getrieben. Ein weiterer Teil der Weizenstärke wird mit der fünffachen Menge Wasser auf dem Wasserbad verkleistert und die obige Pulvermischung mit diesem Kleister angeknetet, bis eine schwach plastische Masse entstanden ist. Die plastische Masse wird durch ein Sieb von etwa 3 mm Maschenweite gedrückt, getrocknet und das trokkene Granulat nochmals durch ein Sieb getrieben. Darauf werden die restliche Weizenstärke, der Talk und das Magnesiumstearat zugemischt und die erhaltene Mischung zu schwach gewölbten Tabletten von je 0,6 g (mit Bruchkerbe) verpresst.

Beispiel 11

Dragées, enthaltend 0,25 g 2-(2-Phenylthioäthyl)-malonsäure-N,N'-diphenyl-monohydrazid bzw. ein Salz davon, z. B. das Natriumsalz, können z. B. folgendermassen hergestellt werden:

1000 g der z. B. nach Beispiel 7 erhältlichen Tabletten werden in üblicher Weise in zwei Stufen mit einem zuckerhaltigen Sirup dragiert. Als solche wird in der ersten Stufe ein Sirup erhalten aus einem Teil Zucker und zwei Teilen Wasser unter Zusatz von Talk (18%), Polyvinylpyrrolidon (1,5%) und Polyäthylenglykol 6000 (1%), und in der zweiten Stufe ein reiner Zuckersirup verwendet.

Beispiel 12

Tabletten, enthaltend 0,1 g Wirkstoff, z. B. 2-(2-Phenylthioäthyl)-malonsäure-N,N'-diphenyl-monohydrazid oder ein Salz, z. B. das Natriumsalz davon, können beispielsweise in folgender Zusammensetzung hergestellt werden:

Zusammensetzung (für eine Tablette)

| | |
|---|---|
| Wirkstoff, z. B. 2-(2-Phenylthioäthyl)-malonsäure-N,N'-diphenyl-monohydrazid | 100 mg |
| Milchzucker | 50 mg |
| Weizenstärke | 73 mg |
| Kolloidale Kieselsäure | 13 mg |
| Talk | 12 mg |
| Magnesium | 2 mg |
| | 250 mg |

Herstellung

Der Wirkstoff wird mit dem Milchzucker, einem Teil der Weizenstärke und mit kolloidaler Kieselsäure gemischt und die Mischung durch ein Sieb getrieben. Ein weiterer Teil der Weizenstärke wird mit der 5fachen Menge Wasser auf dem Wasserbad verkleistert und die Pulvermischung mit diesem Kleister angeknetet, bis eine schwach plastische Masse entstanden ist.

Die Masse wird durch ein Sieb von ca. 3 mm Maschenweite getrieben, getrocknet und das trokkene Granulat nochmals durch ein Sieb getrieben. Darauf werden die restliche Weizenstärke, der restliche Talk und das restliche Magnesiumstearat zugemischt. Die erhaltene Mischung wird zu Tabletten von 0,25 g mit Bruchkerbe(n) verpresst.

Beispiel 13

In analoger Weise wie in den Beispielen 7 bis 12 beschrieben, kann man auch pharmazeutische Präparate, enthaltend eine andere Verbindung der Formel I, vorzugsweise einer der in den Beispielen 1 bis 8 genannten, als Wirkstoff herstellen.

**Patentansprüche**

1. Carbonsäurehydrazid der Formel

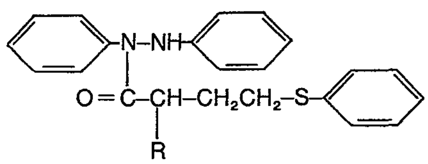

(I),

worin R gegebenenfalls mit einem Alkohol aliphatischen Charakters verestertes Carboxy bedeutet, oder ein pharmazeutisch verwendbares Salz der Verbindung der Formel I, worin R Carboxy darstellt, mit einer Base.

2. Eine Verbindung gemäss Anspruch 1, worin R gegebenenfalls in Salzform vorliegendes Carboxy, Niederalkoxycarbonyl oder Phenylniederalkoxycarbonyl bedeutet, wobei «niedere» Reste bis und mit 7 Kohlenstoffatome aufweisen können.

3. 2-(2-Phenylthioäthyl)-malonsäure-N,N'-diphenyl-monohydrazid oder ein pharmazeutisch verwendbares Salz davon mit einer Base gemäss Anspruch 1.

4. 2-(2-Phenylthioäthyl)-malonsäureäthylester-N,N'-diphenylhydrazid gemäss Anspruch 1.

5. 2-(2-Phenylthioäthyl)-malonsäurebenzylester-N,N'-diphenylhydrazid gemäss Anspruch 1.

6. Verbindungen gemäss einem der Ansprüche 1–5 zur Anwendung in einem Verfahren zur Behandlung des menschlichen oder tierischen Körpers.

7. Eine Verbindung gemäss einem der Ansprüche 1–5 zur Anwendung gemäss Anspruch 6 als anti-thrombotisches Mittel.

8. Pharmazeutische Präparate, enthaltend eine Verbindung gemäss einem der Ansprüche 1–7 neben üblichen Hilfs- und Trägerstoffen.

9. Verfahren zur Herstellung der Carbonsäurehydrazide der Formel

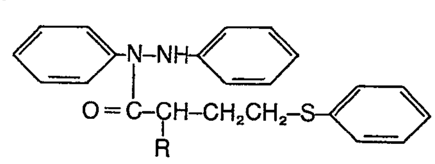

(I),

worin R gegebenenfalls mit einem Alkohol aliphatischen Charakters verestertes Carboxy bedeutet, und von pharmazeutischen verwendbaren Salzen der Verbindung der Formel I, worin R Carboxy darstellt, mit einer Base, dadurch gekennzeichnet, dass man in einer Verbindung der Formel

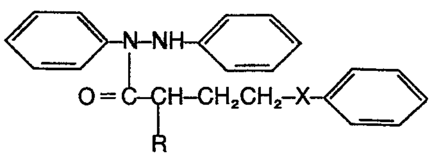

(II),

worin X einen reduktiv in die Thiogruppe überführbaren Rest bedeutet, wobei das sekundäre Stickstoffatom und/oder eine Carboxygruppe R in intermediär geschützter Form vorliegen kann, X zur Thiogruppe reduziert, oder in einer Verbindung der Formel

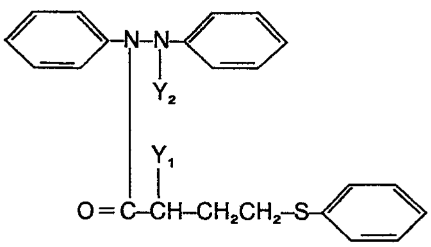

(III) ,

worin $Y_1$ eine funktionell abgewandelte Carboxygruppe und $Y_2$ Wasserstoff oder eine Aminschutzgruppe ist, oder $Y_1$ und $Y_2$ gemeinsam Carbonyl darstellen, die funktionell abgewandelte Carboxygruppe $Y_1$ bzw. unter Spaltung der $Y_2$-N-Bindung die Carbonylgruppe ($Y_1$ + $Y_2$) zu Carboxy solvolysiert, oder Verbindungen der Formeln

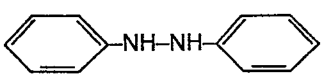

(IV),

und

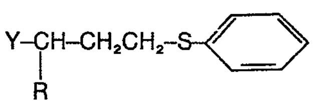

(V),

worin Y eine gegebenenfalls in Anhydridform vorliegende Carboxygruppe bedeutet, und ein Stickstoffatom der Komponente (IV) und/oder eine in der Komponente (V) gegebenenfalls vorhandene Carboxygruppe R in intermediär geschützter Form vorliegen kann, miteinander kondensiert, oder aus einer Verbindung der Formel

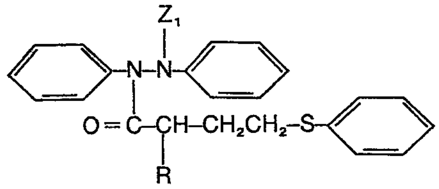

(IV),

worin $Z_1$ einen unter Einführung von Wasserstoff abspaltbaren Rest bedeutet, und eine Carboxygruppe R in intermediär geschützter Form vorliegen kann, $Z_1$ unter Einführung von Wasserstoff abspaltet, oder in einer Verbindung der Formel

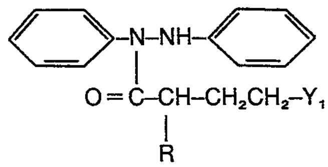

(VII) ,

worin $Y_1$ einen durch Phenylthio ersetzbaren Rest bedeutet, und das sekundäre Stickstoffatom und/oder eine Carboxygruppe R in intermediär geschützter Form vorliegenkann, $Y_1$ durch Phenylthio ersetzt, erforderlichenfalls in der verfahrensgemäss erhältlichen Verbindung die Schutzgruppe(n) entfernt und, wenn erwünscht, die erhaltene Verbindung in eine andere Verbindung der Formel I umwandelt und/oder die gegebenenfalls erhaltene Verbindung der Formel I, worin R Carboxy bedeutet, in ein Salz oder ein erhaltenes Salz einer solchen in die freie Säure oder in ein anderes Salz überführt.

10. Die nach dem Verfahren des Anspruches 9 erhältlichen Verbindungen.

11. Verwendung von Verbindungen gemäss einem der Ansprüche 1–7 zur Herstellung von pharmazeutischen Präparaten.

**Claims**

1. A carboxylic acid hydrazide of the formula

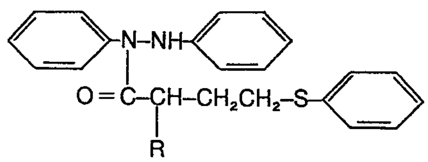

(I) ,

wherein R is carboxy or carboxy esterified with an alcohol of aliphatic character, or a pharmaceutically acceptable salt of a compound of the formula I, wherein R is carboxy, with a base.

2. A compound according to claim 1, wherein R may be carboxy in salt form, lower alkoxycarbonyl or phenyl-lower alkoxycarbonyl, the term «lower» denoting that the radicals so qualified may contain up to and including 7 carbon atoms.

3. 2-(2-Phenylthioethyl)malonic acid N,N′-diphenyl-monohydrazide or a pharmaceutically acceptable salt thereof, with a base, according to claim 1.

4. 2-(2-Phenylthioethyl)malonic acid ethyl ester N,N′-diphenyl hydrazide, according to claim 1.

5. 2-(2-Phenylthioethyl)malonic acid benzyl ester N,N′-diphenyl hydrazide, according to claim 1.

6. A compound according to any one of claims 1 to 5 for use in a method of treating human beings or animals.

7. A compound according to any one of claims 1 to 5 for use according to claim 6 as antithrombotic agent.

8. A pharmaceutical preparation containing a compound according to any one of claims 1 to 7 together with conventional excipients and carriers.

9. A process for the preparation of a carboxylic acid hydrazide of the formula

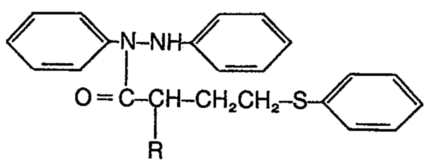

(I),

wherein R is carboxy or carboxy esterified with an alcohol of aliphatic character, or a pharmaceutically acceptable salt of a compound of the formula I, wherein R is carboxy, with a base, which process comprises reducing a radical X to a thio group in a compound of the formula

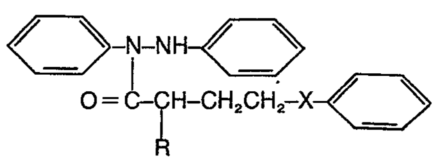

(II),

wherein X is a radical which may be converted by reduction into the thio group, and wherein the secondary nitrogen atom and/or a carboxy group R may be present in temporarily protected form, or, in a compound of the formula

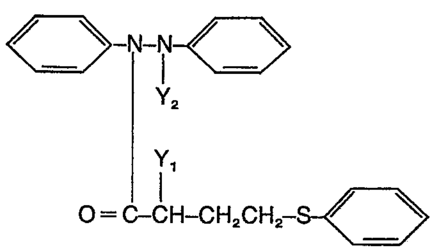

(III),

wherein $Y_1$ is a functionally modified carboxy group and $Y_2$ is hydrogen or an amino protective group, or $Y_1$ and $Y_2$ together are carbonyl, converting the functionally modified carboxy group $Y_1$ or the carbonyl group ($Y_1$ + $Y_2$) by solvolysis to carboxy with removal of the $Y_2$-N bond, or condensing compounds of the formulae

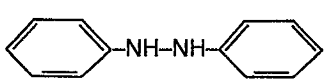

(IV),

and

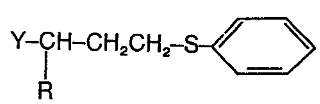

(V),

wherein Y is a carboxy group which may be in anhydride form, and one nitrogen atom of the component (IV) and/or one carboxy group R which may be present in component (V) may be in temporarily protected form, with each other, or eliminating a radical $Z_1$ from a compound of the formula

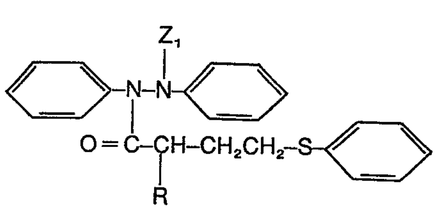

(IV),

wherein $Z_1$ is a radical which may be eliminated by introducing hydrogen, and a carboxy group R may be in temporarily protected form, by introducing hydrogen, or replacing a radical $Y_1$ by phenylthio in a compound of the formula

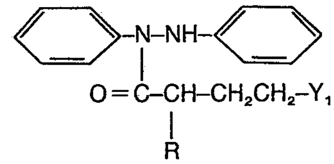

(VII),

wherein $Y_1$ is a radical which may be replaced by phenylthio, and the secondary nitrogen atom and/or a carboxy group R may be in temporarily protected form, if necessary removing the protective group or groups from the compound obtainable by the process of the invention and, if desired, converting the resultant compound into another compound of the formula I and/or converting the resultant compound of the formula I, whenever obtained, wherein R is carboxy, into a salt or a resultant salt of such a compound into the free acid or into another salt.

10. A compound obtainable by the process as claimed in claim 9.

11. Use of a compound according to any one of claims 1 to 7 for the manufacture of pharmaceutical preparations.

**Revendications**

1. Hydrazide d'acide carboxylique de formule

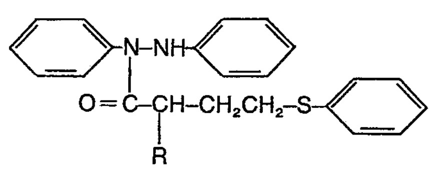

(I)

dans laquelle R représente un groupe carboxy éventuellement estérifié par un alcool à caractère aliphatique, ou un sel acceptable pour l'usage pharmaceutique du composé de formule I dans laquelle R représente le groupe carboxy, et d'une base.

2. Un composé selon la revendication 1, dans lequel R représente un groupe carboxy éventuellement à l'état de sel, un groupe alcoxycarbonyle inférieur ou phényl-(alcoxy inférieur)-carbonyle, les groupes «inférieurs» pouvant contenir jusqu'à 7 atomes de carbone inclus.

3. Le N,N'-diphényl-monohydrazide de l'acide 2-(2-phénylthioéthyl)-malonique ou un sel acceptable pour l'usage pharmaceutique de ce composé et d'une base selon la revendication 1.

4. Le N,N'-diphénylhydrazide du 2-(2-phénylthioéthyl)-malonate d'éthyle selon la revendication 1.

5. Le N,N'-diphénylhydrazide du 2-(2-phénylthioéthyl)-malonate de benzyle selon la revendication 1.

6. Composés selon l'une des revendications 1 à 5, pour l'utilisation dans un procédé pour le traitement de l'organisme humain ou animal.

7. Un composé selon l'une des revendications 1 à 5, pour l'utilisation selon la revendication 6 en tant qu'agent antithrombotique.

8. Compositions pharmaceutiques contenant un composé selon l'une des revendications 1 à 7 avec des produits auxiliaires et véhicules usuels.

9. Procédé de préparation des hydrazides d'acides carboxyliques de formule

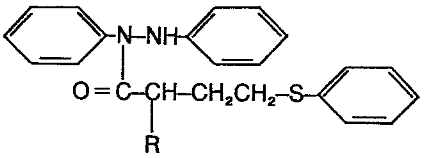

(I)

dans laquelle R représente un groupe carboxy éventuellement estérifié par un alcool à caractère aliphatique, et des sels acceptables pour l'usage pharmaceutique du composé de formule I dans laquelle R représente un groupe carboxy, et d'une base, caractérisé en ce que, dans un composé de formule

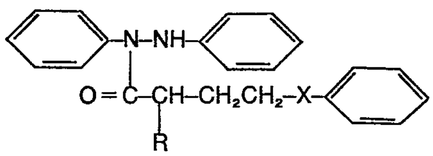

(II)

dans laquelle X représente un reste convertible en le groupe thio par réduction, l'atome d'azote secondaire et/ou un groupe carboxy R pouvant être à l'état provisoirement protégé, on réduit X en groupe thio, ou bien dans un composé de formule

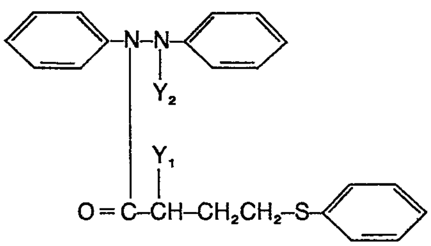

(III)

dans laquelle $Y_1$ représente un groupe carboxy ayant subi une modification fonctionnelle et $Y_2$ représente l'hydrogène ou un groupe protecteur du groupe amino, ou bien $Y_1$ et $Y_2$ représentent ensemble un groupe carbonyle, on solvolyse le groupe carboxy ayant subi une modification fonctionnelle $Y_1$ ou, avec scission de la liaison $Y_2$-N, le groupe carbonyle ($Y_1$ + $Y_2$) en groupe carboxy, ou bien on condense entre eux des composés de formules

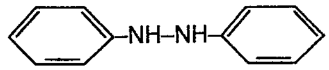

(IV)

et

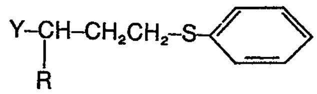

(V)

dans lesquelles Y représente un groupe carboxy éventuellement à l'état d'anhydride, un atome d'azote du composant IV et/ou un groupe carboxy R éventuellement présent dans le composant V peuvent être à l'état provisoirement protégé, ou bien dans un composé de formule

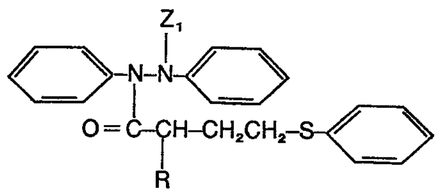

(VI)

dans laquelle $Z_1$ représente un groupe éliminable avec introduction d'hydrogène, et un groupe carboxy R peut être à l'état provisoirement protégé, on élimine $Z_1$ en introduisant de l'hydrogène, ou bien dans un composé de formule

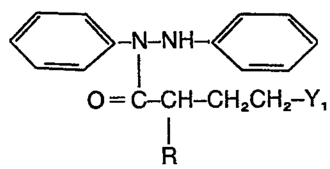

(VII)

dans laquelle $Y_1$ représente un reste remplaçable par le groupe phénylthio, et l'atome d'azote secondaire et/ou un groupe carboxy R peuvent être à l'état provisoirement protégé, on remplace $Y_1$ par un groupe phénylthio, et lorsque c'est nécessaire, dans le composé obtenu ainsi, on élimine le ou les groupes protecteurs et, si on le désire, on convertit le composé obtenu en un autre composé de formule I et/ou on convertit le composé de formule I dans laquelle R représente le groupe carboxy, éventuellement obtenu, en un sel, ou un sel de ce composé, ainsi obtenu, en l'acide libre ou en un autre sel.

10. Les composés qu'on peut obtenir par le procédé de la revendication 9.

11. Utilisation des composés selon l'une des revendications 1 à 7 pour la préparation de compositions pharmaceutiques.